# EUROPEAN PATENT SPECIFICATION

(11) **EP 3 572 514 B1**
(45) Date of publication and mention of the grant of the patent: **12.08.2026**
(21) Application number: 18741098.0
(22) Date of filing: 22.01.2018
(51) Int. Cl.: C07K 14/435, C12N 15/81, A61K 39/002, A61P 33/12

(54) **PRODUCTION OF RECOMBINANT VACCINE AGAINST HELMINTHS IN PICHIA PASTORIS**
HERSTELLUNG EINES REKOMBINANTEN IMPFSTOFFS GEGEN HELMINTHEN IN PICHIA PASTORIS
PRODUCTION D'UN VACCIN RECOMBINANT CONTRE LES HELMINTHS DANS PICHIA PASTORIS

(30) Priority: 23.01.2017 BR 102017001309
(43) Date of publication of application: 27.11.2019
(73) Proprietor: Fundação Oswaldo Cruz, 21045-900 Rio de Janeiro, RJ (BR)
(72) Inventor: SIMPSON, Andrew J G, São Paulo 13287-080 (BR); RAMOS, Celso Raul Romero, Rio de Janeiro - RJ 20510-060 (BR); TENDLER, Miriam, Rio de Janeiro - RJ 22611-100 (BR)
(74) Representative: Witte, Weller & Partner Patentanwälte mbB
(86) International application number: PCT/BR2018/000001
(87) International publication number: WO 2018/132882

(56) References cited:
- EP-A1- 2 617 829
- WO-A2-2004/067698
- BR-A2- PI1 005 855
- MIRIAM TENDLER ET AL: "Development of the Brazilian Anti Schistosomiasis Vaccine Based on the Recombinant Fatty Acid Binding Protein Sm14 Plus GLA-SE Adjuvant", FRONTIERS IN IMMUNOLOGY, vol. 6, 12 May 2015 (2015-05-12), XP055741916, DOI: 10.3389/fimmu.2015.00218
- WU J.M. ET AL: "Combined use of GAP and AOX1 promoter to enhance the expression of human granulocyte-macrophage colony-stimulating factor in Pichia pastoris", ENZYME AND MICROBIAL TECHNOLOGY, vol. 33, no. 4, 1 September 2003 (2003-09-01), US, pages 453 - 459, XP093364602, ISSN: 0141-0229, DOI: 10.1016/S0141-0229(03)00147-9
- ÇALIK, P. ET AL.: "Recombinant protein production in Pichia pastoris under glyceraldehyde-3-phosphate dehydrogenase promoter: From carbon source metabolism to bioreactor operation parameters", BIOCHEMICAL ENGINEERING JOURNAL, vol. 95, 2015, pages 20 - 36, XP029132898, Retrieved from the Internet <URL:https://doi.org/10.1016/j.bej.2014.12.003>
- WATERHAM, H. R. ET AL.: "Isolation of the Pichia pastoris glyceraldehyde-3-phosphate dehydrogenase gene and regulation and use of its promoter", GENE, vol. 186, no. 1, 1997, pages 37 - 44, XP004054877
- MULLER, J. M. ET AL.: "GAP promoter-based fed-batch production of highly bioactive core streptavidin by Pichia pastoris", BIOTECHNOL PROG., vol. 32, no. 4, 18 April 2016 (2016-04-18), pages 855 - 864, XP055619693, ISSN: 8756-7938, DOI: 10.1002/btpr.2283
- VOGL, T. ET AL.: "Regulation of Pichia pastoris promoters and its consequences for protein production", NEW BIOTECHNOLOGY, vol. 30, no. 4, 2013, pages 385 - 404, XP002733016, Retrieved from the Internet <URL:htLps://doi.org/10.1016/j.nbt.2012.1L010>
- ZHANG, A. ET AL.: "Recent advances on the GAP promoter derived expression system of Pichia pastoris", MOLECULAR BIOLOGY REPORTS, vol. 36, no. 6, 2009, pages 1611 - 1619, XP019684802
- VARALDO, P. B. ET AL.: "Mycobacterial Codon Optimization of the Gene Encoding the Sml4 antigen of Schistosoma mansoni in Recombinant Mycobacterium bovis Bacille Calmette-Guerin Enhances Protein Expression but not protection against Cercarial Challenge in mice", FEMS IMMUNOLOGY AND MEDICAL MICROBIOLOGY, vol. 48, no. 1, 2006, pages 132 - 139, XP002721778, ISSN: 0928-8244, DOI: 10.1111/j.1574-695X.2006.00133.x

## Description

### Field of application

The present invention is related to the field of recombinant protein production using a synthetic gene associated with high protein expression in *Pichia pastoris.* More specifically, the invention describes the production of Sm14 *Schistosoma mansoni* recombinant protein, where a synthetic gene was created to promote high expression of such protein, a gene which was cloned under control of two types of *Pichia pastoris* promoters: methanol-inducible promoter (AOX1) and constituent promoter (GAP). With these constructions, *Pichia pastoris* strains were genetically manipulated to efficiently produce vaccine antigen Sm14. The processes to produce and purify this protein from *P*. *pastoris* cells, which can be escalated for their industrial production, were also improved.

### Invention fundamentals

The Sm14 protein which has a molecular weight of approximately 14.8 kDa and belongs to the protein family which binds to fatty acids (Fatty Acid Binding Proteins, FABP), has already been widely studied and described by the applicant in their previous patent applications related to this technical matter.

The three-dimensional structure of protein Sm14 was predicted through molecular modeling by computerized homology, as well as crystallography and Nuclear Magnetic Resonance. The structure of protein Sm14 allowed us to identify potential protective epitopes and enabled us to use rSm14 as a vaccination antigen. This structure, as well as the models built for homologous *Fasciola hepatica* proteins (FABP type 3 being the one which shares greater sequential identity, 49%), shows that these molecules adopt three-dimensional configurations typical of the FABP family members). Protein Sm14 was the first FABP of parasites to be characterized. The scientific literature data indicate the parasites' FABPs as important targets for the development of drugs and vaccines against such infectious agents.

Therefore, based on the entire state-of-the-art of knowledge gathered by the inventors, it will be demonstrated here that protein Sm14 recombinant forms can provide high protection against infections caused by supposedly pathogenic helminths in relation to humans and animals.

In papers which demonstrated the protective activity of Sm14 for the first time, the corresponding recombinant protein was expressed with the pGEMEX-Sm14 vector in the form of inclusion bodies. After the bodies were isolated and washed, the protein was purified by preparative electrophoresis by electroeluting the corresponding band (Tendler et al., 1996). However, this methodology was not appropriate for producing proteins in a larger scale. Later, protein Sm14 started being produced with a fusion of six consecutive histidines (6xHis) in the extreme amino terminal in *Escherichia coli* expression system, in the form of inclusion bodies. After the bodies were obtained and solubilized, refolding was required in order to obtain an immunologically active protein (Ramos *et al., 2001*)*.*

The Brazilian patent application PI 1005855-9 (corresponding to US patents 9.193.772 and US9.475.838 and to EP patent application EP2617829) is related to the obtainment of a synthetic gene for protein Sm14 protein. The genetic transformation of *Pichia pastoris* with such synthetic gene under control of promoter AOX1 allows the production and purification of protein Sm14. The obtainment of protein Sm14 is reached from a synthetic gene, containing optimized codons for high *Pichia pastoris* expression.

Protein Sm14 has also be expressed in Pichia pastoris by use of the GAP promoter (Tendler et al., Front. Immunol. 2015 May 12:6:218).

However, it is noted that despite of the entire knowledge gained by the inventors, there are still disadvantages to be overcome to obtain an antigen material which can be obtained with high yield, in industrial scales under BPF conditions, and which does not lose the stability characteristics.

### Summary of the invention

This invention proposes a platform for producing a recombinant Sm14 vaccine antigen against helminths in *Pichia pastoris.* Through the referred platform, it is possible to obtain a recombinant vaccine against helminths (in *P. pastoris*)*,* including the production and purification processes of protein Sm14 developed in the *Pichia pastoris* system.

The invention uses a synthetic gene for protein Sm14 expression. The genetic transformation of *Pichia pastoris* with such synthetic gene under control of the AOX1 and GAP promoters allows to produce and purify protein Sm14.

Thus, the invention allows to obtain protein Sm14 from a synthetic gene containing codons optimized for high expression in *Pichia pastoris,* SEQ ID NO:3 (final gene sequence), as well as protein Sm14 purification procedures.

### Brief Description of the Figures

Figure 1 shows the strategy for building the pPIC9K-Sm14-MV and pGAP9K-Sm14-MV plasmids.
Figure 2 shows the induction of protein Sm14 expression in *P*. *pastoris* GS115/pPIC9K-Sm14-MV.
Figure 3 shows protein Sm14 expression in *P. pastoris* GSI15/pGAP9K-Sm14-MV.
Figure 4 shows the result of the induction of protein Sm14 expression in *P. pastoris* GS115/pPIC9K-Sm14-MV culture in fermenter.
Figure 5 shows the *western blot* analysis of purified protein of *P*. *pastoris.*
Figure 6 shows the circular dichroism spectrum of *P. pastoris* purified protein Sm14.

### Detailed Description of the Invention

The main purpose of the invention which is to produce a recombinant vaccine against helminths, can be achieved by producing recombinant proteins using a synthetic gene for high protein expression in *Pichia pastoris.* According to the invention a synthetic gene was created to promote high expression of protein Sm14, and with such gene the *Pichia pastoris* strain was obtained and genetically manipulated to effectively produce a vaccine. This invention further contemplates this protein production and purification processes from *P. pastoris* cells; which can be escalated for industrial production.

A *Pichia pastoris* is an efficient host to express and secrete heterologous proteins. The main promoter used for expression in this system is the strongly and firmly regulated promoter from the alcohol oxidase 1 gene (AOX1), inducible by methanol. However, this invention describes a system using an alternative promoter to prevent the use of methanol. The promoter of enzyme glyceraldehyde 3 phosphate dehydrogenase (pGAP) gene was used in the present invention for the constituent expression of the heterologous protein, once this system is more appropriate for large scale production because it eliminates the risk and costs associated with the storage and supply of large volume of methanol.

### 1. OBTAINMENT OF PICHIA PASTORIS RECOMBINANT STRAIN

### 1.1 Synthetic gene for Sm14 expression in P. pastoris:

First a gene was designed and synthesized containing optimized codons to obtain maximum expression of protein Sm14 in *P. pastoris.* In the present case protein Sm14-MV was used; however any form of protein Sm14 can be used.

There is evidence in the literature about the differential use of codons between proteins expressed in low amount and high amount, in the same organism (Roymondal and Sahoo, 2009). However, the codon use tables available in databases (for example: (www.kazusa.or.jp/codon) contain data from all body proteins, without taking the gene expression level into account. For this reason, in order the gene design, a codon use table was first elaborated based on data about sequences that codify recombinant proteins expressed over 1 gram per culture Liter in *P. pastoris* (see Table 1), as well as the sequence for AOX1 protein (which represents 30% of total *P. pastoris* protein, after induction with methanol).

**Table 1: List of high-expression recombinant proteins in P. pastoris.**

| **Expressed protein** | **(mg/L)** | **Reference** |
|---|---|---|
| Hydroxynitrile lyase | 22000 | Hasslacher, M. et al. (1997) Protein Expr. Purif. 11: 61-71 |
| Mouse gelatin | 14800 | Werten, M.W. et al. (1999) Yeast 15: 1087-1096 |
| Tetanus toxin fragment C | 12000 | Clare, J.J. et al. (1991) Bio/Technology 9: 455-460 |
| Human tumor necrosis factor | 10000 | Sreekrishna, K. et al. (1989) Biochemistry 28: 4117-4125 |
| α-amylase | 2500 | Paifer, E. et al. (1994) Yeast 10: 1415-1419 |
| T2A peroxidase | 2470 | Thomas, L. et al. (1998) Can. J. Microbiol. 44: 364-372 |
| Catalase L | 2300 | Calera, J.A. et al. (1997) Infect. Immun. 65: 4718-4724 |
| Hirudin | 1500 | Rosenfeld, S.A. et al. (1996) Protein Expr. Purif. 8: 476-482. |

For gene design, protein Sm14-MV sequence, which has a valine residue at position 62 has been chosen - in place of cystein, which makes it more stable (Ramos *et al.,* 2009); represented here as SEQ ID NO:1.

SEQ ID NO:1
MSSFLGKWKL SESHNFDAVM SKLGVSWATR QIGNTVTPTV TFTMDGDKMT
MLTESTFKNL SVTFKFGEEF DEKTSDGRNV KSVVEKNSES KLTQTQVDPK
NTTVIVREVD GDTMKTTVTV GDVTAIRNYK RLS

After the first selection of codons according to the table created with protein data from Table 1, the clearance of sequences which resulted in transcription termination sites (ATTTA), splicing cryptic donors and receptors (MAGGTRAGT and YYYNTAGC, respectively) and repetitive sequences (cut off sites for restriction enzymes BamHI and EcoRI) was conducted. SEQ ID NO:2 shows the sequence designed to express protein Sm14-MV in *Pichia pastoris.*

SEQ ID NO:2
1 ATGTCTTCTT TCTTGGGTAA GTGGAAGTTG TCTGAATCTC
ACAACTTCGA 51

CGCTGTTATG TCTAAGTTGG GTGTTTCTTG GGCTACCAGA
CAAATTGGTA 101
ACACCGTTAC TCCAACCGTT ACCTTCACCA TGGACGGTGA
CAAGATGACT 151
ATGTTGACCG AGTCTACCTT CAAGAACTTG TCTGTTACTT TCAAGTTCGG
201
TGAAGAGTTC GACGAAAAGA CTTCTGACGG TAGAAACGTT
AAGTCTGTTG 251
TTGAAAAGAA CTCTGAATCT AAGTTGACTC AAACTCAAGT TGACCCAAAG
301
AACACTACCG TTATCGTTAG AGAAGTTGAC GGTGACACTA
TGAAGACTAC 351
TGTTACCGTT GGTGACGTTA CCGCTATCAG AAACTACAAG AGATTGTCTT
401
AA

The Kozak sequence of protein AOX1 gene of *P*. *pastoris* (AAACG) was added to the 5'-end of the designed sequence. Finally, the restriction sites for BamHI (GGATCC) and EcoRI (GAATTC) were added to 5' and 3'-ends of the designed gene, respectively.

SEQ ID NO:3 shows the final sequence of the synthetic gene for protein Sm14 production.

SEQ ID NO:3
1 GGATCCAAAC GATGTCTTCT TTCTTGGGTA AGTGGAAGTT
GTCTGAATCT 51
CACAACTTCG ACGCTGTTAT GTCTAAGTTG GGTGTTTCTT
GGGCTACCAG 101
ACAAATTGGT AACACCGTTA CTCCAACCGT TACCTTCACC
ATGGACGGTG 151
ACAAGATGAC TATGTTGACC GAGTCTACCT TCAAGAACTT GTCTGTTACT
201

TTCAAGTTCG GTGAAGAGTT CGACGAAAAG ACTTCTGACG
GTAGAAACGT 251
TAAGTCTGTT GTTGAAAAGA ACTCTGAATC TAAGTTGACT CAAACTCAAG
301
TTGACCCAAA GAACACTACC GTTATCGTTA GAGAAGTTGA
CGGTGACACT 351
ATGAAGACTA CTGTTACCGT TGGTGACGTT ACCGCTATCA
GAAACTACAA 401
GAGATTGTCT TAAGAATTC

Following the synthesis of the designed sequence (SEQ ID NO:3), the cloning and later sequencing of the synthetic gene was performed in vector pCR2.1 to confirm the fidelity of the synthesized sequence with the designed sequence.

### 1.2 Constructions of plasmids for protein Sm14 expression in Pichia pastoris:

The synthesized gene was cloned in vector pPIC9K where protein Sm14 is expressed without any fusion, allowing its intracellular production.

Vector pPIC9K (Invitrogen) was chosen for the construction of Sm14 expression plasmid in *P. pastoris* for the following reasons:
(1) Possibility for use to express intracellular proteins replacing the alpha-factor gene with the gene of interest, through the vector's BamHI restriction site, located before the Kozak sequence and the beginning of translation. For this purpose, it was necessary to recreate the Kozak sequence before the ATG of the ORF to be expressed, according to Sm14's synthetic gene design.
(2) It has the advantage to allow the selection of clones with multiple copies integrated into the genome, by selecting resistance against antibiotic G418. This possibility did not exist with pPIC9, previously used.

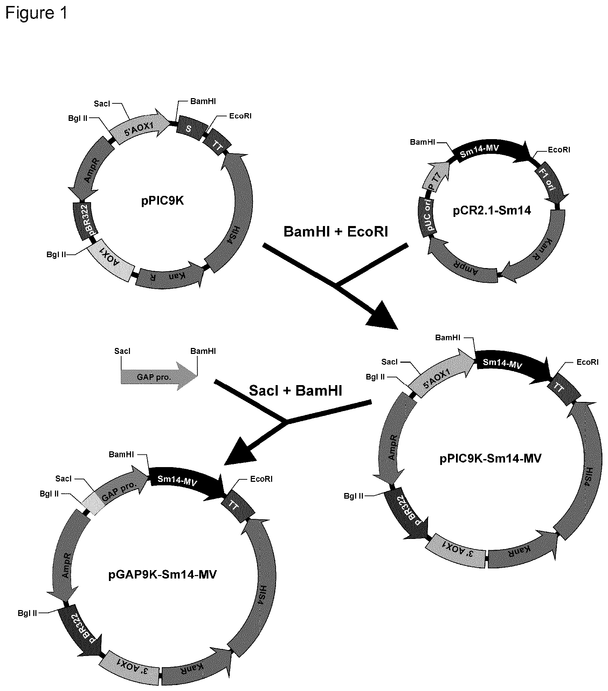

The strategy for building plasmid pPIC9K-Sm14 is described in Figure 1. According to this strategy, plasmid pCR21-Sm14-MV and vector pPIC9K were digested with restriction enzymes BamHI and EcoRI. After digestion, the DNA fragments were separated by agarose gel electrophoresis and the fragments corresponding to vector pPIC9K and to the synthetic Sm14-MV insert were excised from the agarose gel, purified and bond using T4 DNA ligase. *E.coli's* DH5α strain was transformed by link reaction and the clones were selected in LB agar medium containing ampicillin. The pDNA of some ampicillin-resistant clones was purified and analyzed by restriction with enzymes BamHI and EcoRI, as well as by DNA sequencing. The construction so obtained was called pPIC9K-Sm14-MV, which expresses protein Sm14 under control of the strong alcohol oxidase 1 (AOX1) promoter, which is induced by methanol (Cregg *et al.,* 1993).

According to the present invention, another promoter was successfully used in the expression of recombinant proteins in *Pichia pastoris,* the constituent promoter of glyceraldehyde-3-phosphate dehydrogenase (GAP) gene. For the large scale production of recombinant proteins, the promoter GAP-based expression system is more appropriate than promoter AOX1, because the risks and costs associated with the transport and storage of large volume of methanol are eliminates (Zhang *et al,* 2009).

According to the strategy described in Figure 1, promoter GAP was amplified from the genomic DNA of strain GS115 of *Pichia pastoris,* using primers GAP-for (SEQ ID NO: 4) and GAP-rev (SEQ ID NO:5), which contain the restriction sites Sacl and BamHI, respectively.
**SEQ ID NO:4**
   GAGCTCTTTT TTGTAGAAAT GTCTTGG 27
**SEQ ID NO:5**
   GGATCCAAAT AGTTGTTCAA TTGATTGAAA TAGG 34

With these oligonucleotides, a fragment of 506 pairs of bases corresponding to promoter GAP of *Pichia pastoris* was amplified (SEQ ID NO: 6).

SEQ ID NO:6
GAGCTCTTTT TTGTAGAAAT GTCTTGGTGT CCTCGTCCAA TCAGGTAGCC
50

ATCTCTGAAA TATCTGGCTC CGTTGCAACT CCGAACGACC
TGCTGGCAAC 100
GTAAAATTCT CCGGGGTAAA ACTTAAATGT GGAGTAATGG
AACCAGAAAC 150
GTCTCTTCCC TTCTCTCTCC TTCCACCGCC CGTTACCGTC
CCTAGGAAAT 200
TTTACTCTGC TGGAGAGCTT CTTCTACGGC CCCCTTGCAG
CAATGCTCTT 250
CCCAGCATTA CGTTGCGGGT AAAACGGAGG TCGTGTACCC
GACCTAGCAG 300
CCCAGGGATG GAAAAGTCCC GGCCGTCGCT GGCAATAATA
GCGGGCGGAC 350
GCATGTCATG AGATTATTGG AAACCACCAG AATCGAATAT
AAAAGGCGAA 400
CACCTTTCCC AATTTTGGTT TCTCCTGACC CAAAGACTTT AAATTTAATT
450
TATTTGTCCC TATTTCAATC AATTGAACAA CTATTTTTGA ACAACTATTT
500
GGATCC 506

The amplified promoter GAP's DNA, as well as plasmid pPIC9K-Sm14-MV was digested with restriction enzymes Sacl and BamHI. The fragments corresponding to vector pPIC9K-SM14-MV and to insert GAP were purified from agarose gel, purified and bonded. E.coli's DH5α strain was transformed by link reaction and the clones were selected in LB agar medium containing ampicillin. The pDNA of some ampicillin-resistant clones was purified and analyzed by restriction with enzymes BamHI and EcoRI, as well as by DNA sequencing. The construction so obtained was called pGAP9K-Sm14-MV, which expresses the protein under control of constituent promoter pGAP.

### 1.3 Transformation of P. pastoris with plasmids pPIC9K-Sm14-MV and pGAP9K-Sm14-MV. And selection of recombinant clones with multiple copies

In order to produce the protein, the GS115 (*his4*) of *P. pastoris* strain was transformed with plasmids pPIC9K-Sm14-MV and pGAP9K-Sm14-MV, digested with enzymes Sacl. With the DNA digested and purified, the competent cells for electroporation of strain GSI15 were transformed.

After transformation, the cells were spread in RD medium (histidine-free medium, which contains: 1 M sorbitol; 2% dextrose; 1.34% YNB; 4x10⁻⁵% biotin; and 0.005% of each amino acid: L-glutamate, L-methionine, L-lysine, L-leucine and L-isoleucine for the selection of strains transformed by auxotrophy marker *his4.* Clones that managed to grow in the histidine-free medium were submitted to selection with antibiotic G418, at the concentrations of 0.5; 1; 2; and 4 mg/ml, in a YPD culture (1% Yeast Extract, 2% Peptone; 2% dextrose) at 30°C.

In order to confirm whether the selected clones presented the expression cassette of the synthetic gene, genomic DNA of the clones was purified and used in PCR reactions with primers AOX5' and AOX3' (for pPIC9K-Sm14-MV) and GAP5' and AOX3' (for pGAP9K-Sm14-MV).

### 1.4 Cultivation expression in shaker

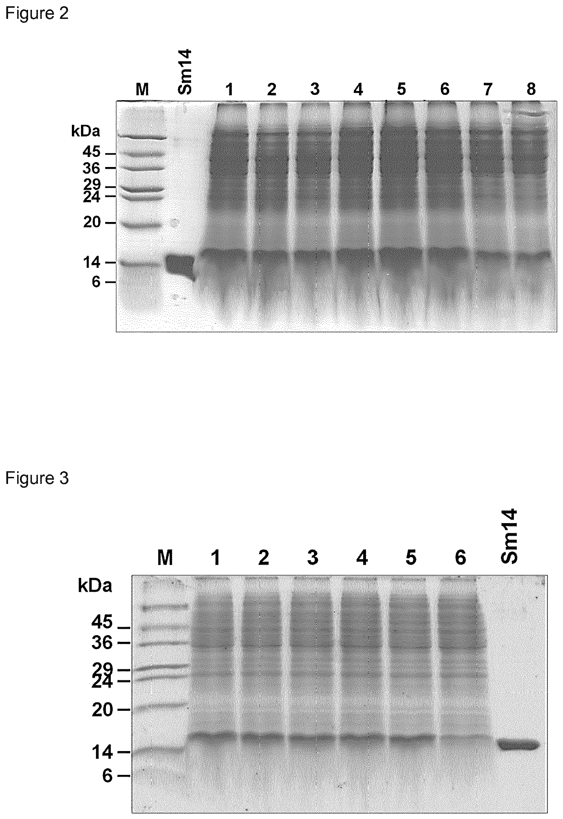

In order to test the expression of protein Sm14 in *P*. pastoris/pPIC9K-Sm14-MV clones, the clones were grew in shaker in 5 mL of the BMG medium (Buffered Minimal Glycerol medium, containing: 1.34% YNB; 0.04% biotin, 0.1 M potassium phosphate pH 6.0; and 1% glycerol) at 30°C for 48 hours and then transferred to a BMM medium, 5 ml, (Buffered Minimal Methanol medium, containing the same components as the BMG medium, except for glycerol which was replaced with 0.5% methanol), for the induction of recombinant protein expression. After 72 hours, adding 0.5% methanol every 24 hours, the total proteins of each clone were analyzed by SDS-PAGE (Figure 2). Figure 2 shows the result of inducing the expression of protein Sm14 in *P*. *pastoris* clones GS115/pPIC9K-Sm14-MV.
**M.-** Low Molecular Weight Marker
**Sm14.-** Control of protein Sm14 without purified fusion of *E. coli*
**1 to 8.-** Total protein of clones 1 - 17 GS115/pPIC9K-Sm14-MV after induction with methanol.

In order to test the expression of protein Sm14 in *P*. pastoris/pGAP9K-Sm14-MV clones, the clones were grown in 5 ml of the BMG medium for only 72 hours. Figure 3 shows the result of protein Sm14 expression in *P. pastoris* GS115/pGAP9K-Sm14-MV clones.
**M.-** Low Molecular Weight Marker
**1 to 6.-** Total protein of clones 1 - 17 GSI15/pGAP9KSm14-MV following 3 days of culture in BMG medium.
**Sm14.-** Control of protein Sm14 without purified fusion of *E. coli*

In all the selected clones, it was possible to note a majority band which coincides with the size of purified *E. coli* purified fusionless Sm14 protein.

### 1.5 Expression of Sm14 recombinant in P. pastoris GS115/ pPIC9K-Sm14-MV in fermenter.

Fermentation was conducted with a 5-Liter culture in a fermenter with automatic supply, pH, antifoaming adjustments. The fermentation medium used was Basal Salt Medium (BSM) supplemented with the metal salt solutions (trace elements) and biotin.

In order to prepare the inoculum, an ampoule from the working bank of *Pichia pastoris* GS115/pPIC9K-Sm14-MV strain was used to inoculate the YDP medium and cultivated in shaker for 18 hours at 220 rpm, 30°C. Subsequently, the cells are inoculated in BSM medium supplemented with glycerol, cultivate in shaker for 18 hours before inoculating the fermenter.

The first fermentation phase (Glycerol fed batch) is started by adding 50% glycerol containing trace elements (12%) to the culture. The temperature and pH were maintained at 30°C and 5.00, respectively and aeration in 1.0 VVM.

Induction was starting by adding 100% methanol, containing trace elements (12%) to the fermenter. Following the adaptation period, methanol was added according to the dissolved oxygen behavior.

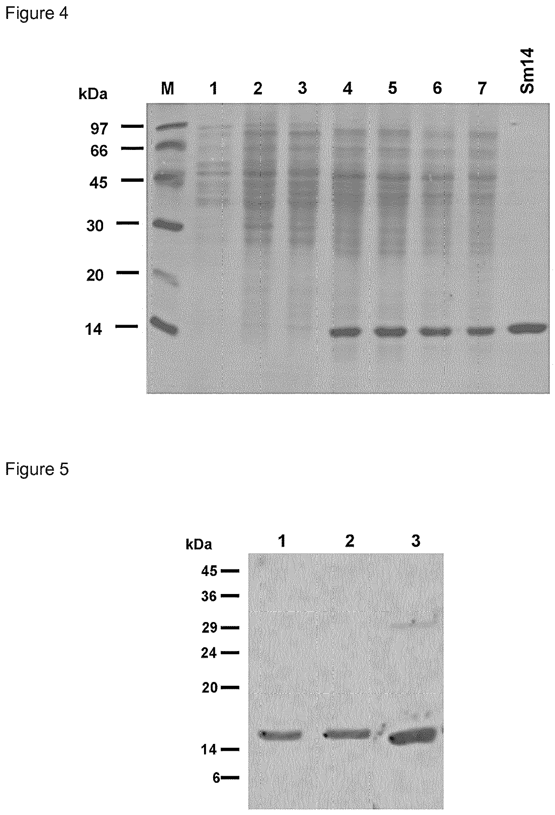

Figure 5 shows the induction of protein Sm14 expression in *P*. *pastoris* GS115/pPIC9K-Sm14-MV, in fermenter, where:
**M.-** Low Molecular Weight Marker
**1-7.-** Induction of expression for 24, 48, 72 and 96, 120, 144 and 168 hours, respectively.
**Sm14.-** Control of protein Sm14 without purified fusion of *E. coli*

Thus, the recombinant protein-specific induction corresponding to Sm14 in *P. pastoris* in the cultivation in fermentation was proven.

The yield of wet molecular mass reached of 170 g per liter and the estimated yield of Sm14 is of approximately 1 g of protein per culture liter. By changing the fermentation conditions, it is possible to exceed the cell mass yield and, correspondently the yield obtained of protein Sm14.

Even so, the obtained value already corresponds to a high protein Sm14 expression level, which comprises the majority protein of the cells following induction with methanol.

### 2. PURIFICATION OF RECOMBINANT PROTEIN SM14 EXPRESSED IN P. pastoris GSI15/pPIC9K-Sm14-MV STRAIN

The purification protocol for recombinant protein Sm14 from *P*. *pastoris* cytoplasm was based on the methodology developed at the Experimental Schistosomiasis Laboratory of the Oswaldo Cruz Institute (IOC) for Sm14 purification without fusion into the *E. coli* system.

**Lysis:** The purification of recombinant protein starts with the lysis of *P. pastoris* cells. For that purpose, the cells are resuspended 30mM Tris-HCI 30mM pH 9.5 and lysed through pressure at 1500 Bar. The lysate is clarified by centrifugation.

**Conditioning:** The clarified lysate is submitted to tangential filtration in the 100 kDa membrane in order to remove high molecular weight proteins, followed by concentration and diafiltration in the 3 kDa membrane with buffer 30mM Tris-HCI 30mM pH 9.5 until conductivity corresponds to the buffer value.

**Capture:** Clarified lysate is loaded in resin Q-Sepharose XL (GE Healthcare) resin, balanced with buffer A (30 mM Tris-HCI pH 9.5). Then, the culture is washed with buffer A, and the protein is eluted with buffer B (30 mM Tris-HCI pH 8.0) where protein Sm14 elutes with a higher purity degree.

**Polishing:** The eluted protein of resin Q-sepharose XL presents few contaminant proteins. In order to separate such proteins from protein Sm14, the gel-filtration in resin Sephacryl S100 HR (GE Healthcare) was used, using PBS pH 7.4 as mobile phase.

### 4. ANALYSIS OF RECOMBINANT PROTEIN Sm14 PRODUCED IN Pichia pastoris

The recombinant protein was purified from *P. pastoris* by using the same physical-chemical characteristics as protein Sm14-MV without fusion expressed in *E. coli.* The protein so purified from *P. pastoris* corresponds in size with the protein specifically induced by methanol. Since we have the synthetic gene of protein Sm14-MV under control of AOX1 promoter in the expression cassette, we deduce that the expressed and purified *P. pastoris* protein is protein Sm14-MV.

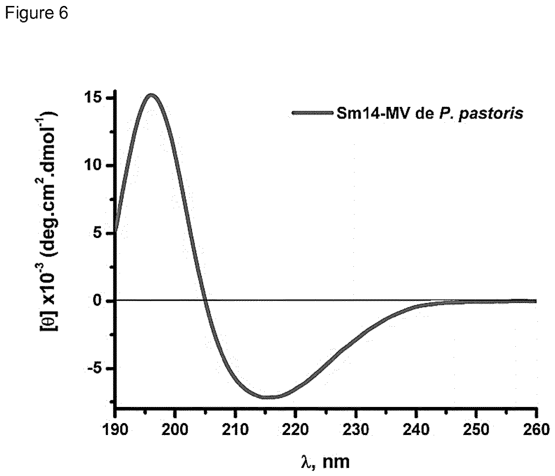

In order to confirm this statement, *P. pastoris'* purified protein was analyzed by western blot, by using rabbit anti-Sm14 serum (Figure 6). Figure 6 represents the western blot analysis of *P*. *pastoris'* purified protein.
**1 and 2** - Protein Sm14-MV purified from *P. pastoris*
**3.-** Positive control of protein Sm14 without purified fusion of *E. coli*

In this experiment, it was possible to observe that anti-Sm14 antibodies specifically recognized *P. pastoris'* purified protein (rabbit serum does not recognize endogenous *P. pastoris* proteins, data not shown), therefore confirming its identity.

Finally, it was necessary to identify whether the *P. pastoris* purified protein has a structure which corresponds to beta folding, characteristic of proteins of the Fatty Acid Binding Protein family, to which Sm14 belongs. To do so, protein samples were analyzed by circular dichroism, using spectral photopolarimeter J-815 (JASCO) (Figure 7). Figure 9 shows the circular dichroism spectrum of *P. pastoris'* purified protein Sm14.

As it can be noted in Figure 7, the spectrum corresponds to a beta-structure protein. This spectrum was similar to circular dichroism spectra of lots of protein Sm14 previously purified from *E. coli* in laboratory.

Thus, based on the report above we may conclude that this invention allows us to:
- design and synthesize a synthetic gene for high expression of protein Sm14-MV in *Pichia pastoris;*
- build pPIC9K-Sm14-MV and pGAP9K-Sm14-MV expression plasmids containing the synthetic gene's sequence under control of AOX1 and GAP promoters, respectively;
- obtain *P. pastoris* strains producing protein Sm14; and,
- purify protein Sm14 in two chromatographic steps, feasible for scheduling for industrial production.

Through the process of the present invention, it was possible to build the pPIC9K-Sm14-MV and pGAP9K-Sm14MV plasmids for the constituent expression of protein Sm14 in *Pichia pastoris* and select the producing clones from the transformation of strain GS115 with plasmid pGAP9K-Sm14MV. One of the advantages of the invention is that the strains produce the protein within a lower time compared to the methanol induction system in constituent way. In addition, the new strain simplifies the fermentation process for the production of protein Sm14.

Therefore, the present invention described herein shows that protein Sm14 provides protection against *Schistosoma mansoni* infection in mice, in *E. coli* and *P. pastoris* platforms.

### REFERENCES

(1) Cregg, J.M., Vedvick, T.S. and Raschke, W.C.. Recent advances in the expression of foreign genes in Pichia pastoris. BioTechnology v. 11, p. 905-910, 1993.
(2) Faber, K.N., Harder, W., and Veenhuis, M. Review: Methylotropic Yeasts as Factories for the Production of Foreign Proteins. Yeast. v. 11, p. 1331-1344, 1995.
(3) Ramos, C.R., Spisni, A., Oyama, S.Jr., Sforça, M.L., Ramos, H.R, Vilar, M.M., Alves, A.C., Figueredo, R.C., Tendler, M., Zanchin, N.I., Pertinhez, T.A., Ho, P.L. Stability improvement of the fatty acid binding protein Sm14 from S. mansoni by Cys replacement: structural and functional characterization of a vaccine candidate. Biochim Biophys Acta. v. 1794, p. 655 - 662, 2009.
(4) Ramos, C.R., Vilar, M.M., Nascimento, A.L., Ho, P.L., Thaumaturgo, N., Edelenyi, R., Almeida, M., Dias, W.O., Diogo, C.M., Tendler, M. r-Sm14 - pRSETA efficacy in experimental animals. Mem Inst Oswaldo Cruz.v. 96, p.131 - 135, 2001.
(5) Roymondal, U.D. and Sahoo, S.S. Predicting gene expression level from relative codon usage bias: an application to Escherichia coli genome. DNA Res. v. 16, p. 13 - 30, 2009.
(6) Tendler, M., Brito, C.A., Vilar, M.M., Serra-Freire, N., Diogo, C.M., Almeida, M.S., Delbem, A.C., Da Silva, J.F., Savino, W., Garratt, R.C., Katz, N., Simpson, A.S. A Schistosoma mansoni fatty acid-binding protein, Sm14, is the potential basis of a dual-purpose anti-helminth vaccine. Proc Natl Acad Sci U S A. v. 93, p. 269 - 273, 1996.
(7) Zhang, A.L., Luo, J.X., Zhang, T.Y., Pan, Y.W., Tan, Y.H., Fu, C.Y., Tu, F.Z. Recent advances on the GAP promoter derived expression system of Pichia pastoris. Mol Biol Rep. v. 36, p. 1611-1619. 2009.

## Claims

1. Production process of recombinant protein Sm14 of *Schistosoma mansoni* in *Pichia pastoris,* **characterized by**:
(a) synthesizing the gene defined by SEQ ID NO:3;
(b) cloning of the synthesized gene in vector pPIC9K by BamHI site and reconstituting the Kozak sequence of gene AOX1 before the start codon of protein Sm14, for the expression of protein Sm14 in its intracellular form, induced by methanol, wherein the resulting plasmid is pPIC9K-Sm14-MV;
(c) replacement of promoter AOX1 by promoter gene GAP in plasmid pPIC9K-Sm14-MV, with the Sacl and BamHI sites, for the constituent expression of protein Sm14;
(d) transformation of *P. pastoris* with plasmids pPIC9K-Sm14-MV and pGAP9K-Sm14-MV, and selection of recombinant clones with multiple copies.

## Patentansprüche

1. Verfahren zur Herstellung von rekombinantem Sm14-Protein von *Schistosoma mansoni* in *Pichia pastoris,* das folgende Schritte aufweist:
(a) Synthetisieren des durch SEQ ID NO:3 definierten Gens;
(b) Klonieren des synthetisierten Gens in den Vektor pPIC9K über die BamHI-Stelle und Rekonstituieren der Kozak-Sequenz des AOX1-Gens vor dem Startcodon des Sm14-Proteins zur Expression des Sm14-Proteins in seiner intrazellulären Form, induziert durch Methanol, wobei das resultierende Plasmid pPIC9K-Sm14-MV ist;
(c) Ersetzen des Promotors des AOX1-Gens durch den Promotor des GAP-Gens im Plasmid pPIC9K-Sm14-MV unter Verwendung der Sacl- und BamHI-Stellen zur konstitutiven Expression des Sm14-Proteins;
(d) Transformieren von *P. pastoris* mit den Plasmiden pPIC9K-Sm14-MV und pGAP9K-Sm14-MV sowie Selektieren rekombinanter Klone mit mehreren Kopien.

## Revendications

1. Procédé de production de la protéine recombinante Sm14 de *Schistosoma mansoni* dans *Pichia pastoris,* **caractérisé par :**
(a) la synthétisation du gène défini par SEQ ID NO :3 ;
(b) le clonage du gène synthétisé dans le vecteur pPIC9K par le site BamHI et la reconstitution de la séquence Kozak du gène AOX1 avant le codon de départ de la protéine Sm14, pour l'expression de la protéine Sm14 sous sa forme intracellulaire, induite par le méthanol, dans lequel le plasmide résultant est pPIC9K-Sm14-MV ;
(c) le remplacement du promoteur AOX1 par le gène promoteur GAP dans le plasmide pPIC9K-Sm14-MV, avec les sites Sacl et BamHI, pour l'expression constitutive de la protéine Sm14 ;
(d) la transformation de *P. pastoris* avec les plasmides pPIC9K-Sm14-MV et pGAP9K-Sm14-MV, et la sélection de clones recombinants avec des copies multiples.
